# EUROPÄISCHE PATENTANMELDUNG

(11) **EP 1 621 231 A1**
(43) Veröffentlichungstag der Anmeldung: **01.02.2006**
(21) Anmeldenummer: 05015175.2
(22) Anmeldetag: 13.07.2005
(51) Int. Cl.: A61Q 5/00, A61Q 5/12, A61Q 19/10, A61K 8/39, A61K 8/37, A61K 8/365

(54) **Konditionierende tensidische Zubereitung**

(30) Priorität: 22.07.2004 DE 102004035633
(71) Anmelder: Cognis IP Management GmbH, 40589 Düsseldorf (DE)
(72) Erfinder: Köster, Josef, Harleysville PA 19438 (US); Schmid, Karl Heinz, 40822 Mettmann (DE); Doerr, Markus, Glenmoore PA 19343 (US); Behler, Ansgar, 46240 Bottrop (DE); Hensen, Hermann, 42781 Haan (DE); Seipel, Werner, 40723 Hilden (DE); Hüttner, Iris, 47906 Kempen-Tönisberg (DE); Cornelsen, Sybille, 40883 Ratingen (DE)

(57) **Zusammenfassung**

Vorgeschlagen wird eine kosmetische Zubereitung enthaltend
(a) 0,1 bis 30 Gew.% mindestens eines anionischen, nichtionischen und/oder amphoteren Tensids,
(b) 0,05 bis 7 Gew.% eines α-Hydroxycarbonsäureesters ethoxylierter Alkohole der Formel (I)

   R¹O(CH₂CH₂O)ₙOH (I),

   in der R1 für einen linearen oder verzweigten Alkyl- oder Alkenylrest mit 6 bis 22 Kohlenstoffatomen und n für Zahlen von 1 bis 50 steht,
(c) 0,01 bis 1,5 Gew.% mindestens eines Erdalkaliions und
(d) 0,1 bis 2 Gew.% einer Silikonverbindung sowie die Verwendung von α-Hydroxycarbonsäureester ethoxylierter Alkohole als Konditioniermittel.

## Beschreibung

### Gebiet der Erfindung

Die Erfindung befindet sich auf dem Gebiet der konditionierenden tensidischen Zubereitungen und die Verwendung von langkettigen alkoxylierten Hydroxycarbonsäureestern als Konditionierungsmittel.

### Stand der Technik

Unter Haut- und Haarkonditioniermitteln versteht man üblicherweise Mittel, die die Eigenschaften von Haut und Haar im Sinne des Anwenders verbessern. Hierzu zählt die Verbesserung des Griffes oder des Glanzes von Haar, ebenso die Weichheit von Haut und Haar. Besonders erwünscht ist eine Glättung von Haut und Haar. In tensidischen Formulierungen werden in der Regel kationische Polymere eingesetzt, die diese Wirkung auf Haut und Haar entfalten. Gegenstand der US 6264931 sind beispielsweise 2-in-1-Konditioniermittel enthaltend anionische Tenside in Kombination mit kationischen Polymeren. Diese kationischen Konditioniermittel weisen häufig den Nachteil der schlechten biologischen Abbaubarkeit auf. Außerdem lassen sich pulverförmige Polymere häufig nur sehr schwer homogen in die kosmetischen Zubereitungen einarbeiten.

Tensidische Zubereitungen sollen aber auch eine geringe Haut- und Haarbelastung aufweisen. So beschreibt die EP 0371339 B1 oberflächenaktive wäßrige Mischungen enthaltend Fettalkoholethersulfate und Fettalkoholethercitrate in Kombination mit Erdalkaliionen, die Haut und Haar vergleichsweise wenig belasten.

Aufgabe der vorliegenden Patentanmeldung war es daher Haut- und Haarkonditioniermittel bereitzustellen, die über hervorragenden konditionierende Eigenschaften verfügen, dabei aber extrem haut- und haarschonend sind. Außerdem soll möglichst auf kationische Konditioniermittel verzichtet werden.

### Besehreibunp, der Erfindung

Gegenstand der vorliegenden Erfindung sind kosmetische Zubereitungen enthaltend
(a) 0,1 bis 30 Gew.% mindestens eines anionischen, nichtionischen und/oder amphoteren Tensids,
(b) 0,05 bis 20 Gew.% eines α-Hydroxycarbonsäureesters ethoxylierter Alkohole der Formel (I)

   R¹O(CH₂CH₂O)ₙH (I),

   in der R1 für einen linearen oder verzweigten Alkyl- oder Alkenylrest mit 6 bis 22 Kohlenstoffatomen und n für Zahlen von 1 bis 50 steht,
(c) 0,005 bis 1,5 Gew.% mindestens eines Erdalkaliions und
(d) 0,1 bis 3 Gew.% einer Silikonverbindung.

### Tenside

Als Komponente (a) der kosmetischen Zubereitungen gemäß der vorliegenden Erfindung kommen anionische, nichtionische und/oder amphotere/zwitterionische Tenside in Betracht. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)-sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Alkylglucosecarboxylate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

In einer bevorzugten Ausführungsform werden als Komponente (a) Alkylethersulfate, Alkylamidobetaine, acylierte Aminosäuren, Alk(en)yloligoglycoside, Alkylglucosecarboxylate oder Alkylamphoacetate eingesetzt.

Die Tenside werden in den erfindungsgemäßen Zubereitungen in Mengen von 0,1 bis 20 Gew.%, bevorzugt von 9 bis 20 Gew. % eingesetzt.

### α-Hydroxycarbonsäureester

α-Hydroxycarbonsäuren sind organische Säuren, die neben mindestens einer COOH-Gruppe im Molekül mindestens eine OH-Gruppe enthalten. Sie können als Monohydroxycarbonsäuren mit einer OH-Gruppe vorliegen, mit zwei als Di-, oder mit mehr als zwei OH-Gruppen als Polyhydroxycarbonsäuren vorliegen. Nach der Stellung der OH-Gruppe zur COOH-Gruppe unterscheidet man alpha-, beta-, und gamma-Hydroxycarbonsäuren.

In der vorliegenden Erfindung bevorzugte α-Hydroxycarbonsäuren sind die Weinsäure, Mandelsäure, Milchsäure, Äpfelsäure, Citronensäure und deren Salze sowie deren Eigenkondensationsprodukte. Besonders bevorzugt im Sinne der vorliegenden Erfindung ist die Citronensäure.

Die α-Hydroxycarbonsäureester leiten sich ab von ethoxylierten Alkoholen mit 6 bis 22 Kohlenstoffatomen der allgemeinen Formel (I)

R¹O(CH₂CH₂O)nH (I)

in der R¹ für einen linearen oder verzweigten Alkyl- und/oder Alkenylrest mit 6 bis 22 Kohlenstoffatomen und n für Zahlen von 1 bis 50 steht. Bevorzugt steht in der Formel (I) der Ethoxylierungsgrad n für Zahlen von 1 bis 20, vorzugsweise 1 bis 10 und insbesondere 3 bis 8. Insbesondere geeignet sind Hydroxycarbonsäureester, die sich ableiten von ethoxylierten Alkoholen der Formel (I), in der R¹ für einen linearen Alkylrest steht.

Typische Beispiele sind die Addukte von durchschnittlich 1 bis 20, vorzugsweise 1 bis 10 und insbesondere 3 bis 8 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Addukte von 1 bis 10, insbesondere 3 bis 8 Mol Ethylenoxid an technische Fettalkohole mit 12 bis 18 Kohlen stoffatomen, wie beispielsweise Kokos-, Palm-, Palmkem- oder Talgfettalkohol.

Einer Ausführungsform der vorliegenden Erfindung entsprechend leitet sich R¹O in Formel (I) ab von einer Fettalkoholmischung enthaltend 65 - 75 Gew.% C12-, 20 bis 30 Gew.% C14-, 0- 5 Gew.% C16- und 0 bis 5 Gew.% C18- Alkohole. Diese Alkoholmischung ist kommerziell erhältlich, beispielsweise als Lorol® Spezial, ein Handelsprodukt der Cognis Deutschland GmbH & Co. KG. Hydroxycarbonsäureester auf Basis dieser Fettalkoholmischung weisen vorzugsweise einen Ethoxylierungsgrad n von durchschnittlich 4 auf.

Einer weiteren Ausführungsform der vorliegenden Erfindung entsprechend leitet sich R¹O in Formel (I) ab von einer Fettalkoholmischung enthaltend 45 - 60 Gew. % C12-, 15 - 30 Gew. % C14-, 5 - 15 Gew. % C16- und 8 - 20 Gew. % C 18- Alkohol. Diese Alkoholmischung ist ebenfalls kommerziell erhältlich, beispielsweise als Lorol® technisch, ein Handelsprodukt der Cognis Deutschland GmbH & Co. KG. Hydroxycarbonsäureester auf Basis dieser ausgewählten Fettalkoholmischung sind vorzugsweise mit durchschnittlich 7 Mol Ethylenoxid ethoxyliert worden (n =7).

Im Sinne der vorliegenden Erfindung können die α-Hydroxycarbonsäuren vollständig oder insbesondere partiell verestert sein. Bei der partiellen Veresterung handelt es sich um Verbindungen, die noch mindestens eine freie Carboxylgruppe tragen. Dem entsprechend kann es sich um saure Ester oder deren Neutralisationsprodukte handeln. Vorzugsweise liegen die Partialester in Form der Alkali-, Erdalkali-, Ammonium-, Alkylammonium-, Alkanolammonium- und/oder Glucammoniumsalze vor.

Besonders bevorzugt im Sinne der vorliegenden Erfindung sind als Komponente (b) Ester der Citronensäure mit ethoxylierten Alkoholen der Formel (I)

R¹O(CH₂CH₂O)ₙH (I)

in der R1 für eine linearen oder verzweigten Alkyl- oder Alkenylrest mit 6 bis 22 Kohlenstoffatomen und n für Zahlen von 1 bis 50 steht.

Im Zusammenhang mit den ganz besonders bevorzugten Citronensäureestern handelt es sich vorzugsweise um Mischungen von isomeren Verbindungen der allgemeinen Formel (II) 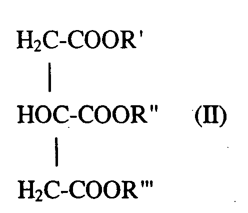
in der R', R", R"' für X und/oder einen ethoxylierten Alkylrest R1 mit der in Formel (I) angegebenen Bedeutung steht, wobei die Verteilung der Reste R', R" bzw. R'" mit der Maßgabe erfolgt, dass das Gewichtsverhältnis von Monoester : Diester im Bereich von 3 : 1 bis 10 : 1 liegt, vorzugsweise liegt das Gewichtsverhältnis von Monoester : Diester im Bereich von 5 : 1 bis 8 : 1.

Die erfindungsgemäß bevorzugten Citronensäureestermischungen enthalten somit zwingend Mono- und Diester, vorzugsweise in Mengen von 50 bis 90 Gew.%, insbesondere von 60 bis 80 Gew.% - berechnet als Mono- und Diester und bezogen auf Mischung. Die Mischungen können als den zu 100 Gew. % fehlenden Rest noch Triester und freie Citronensäure enthalten. Vorzugsweise enthalten die Mischungen aber wenig freie Citronensäure, wobei weniger als 10 Gew. % - bezogen auf Mischungen - bevorzugt sind.

Somit stellen die erfindungsgemäß bevorzugten Citronensäureester hauptsächlich Partialester der Citronensäure dar, die noch mindestens eine freie Carboxylgruppe enthalten. Dementsprechend kann es sich auch um saure Ester oder deren Neutralisationsprodukte handeln, und X kann in Formel (II) für Wasserstoff oder ein Kation stehen. Vorzugsweise liegen die Partialester dann in Form von Alkali-, Erdalkali-, Ammonium-, Alkylammonium-, Alkanolammonium- und/oder Glucammoniumsalze vor (d.h. X steht für Alkali-, Erdalkali-, Ammonium-, Alkylammonium-, Alkanolammonium- und/oder Glucammonium-Ion).

Die α-Hydroxycarbonsäureester ethoxylierter Alkohole der Komponente (b) der vorliegenden Erfindung werden in den kosmetischen Zubereitungen in Mengen von 0,05 bis 20 Gew. %, bevorzugt von 0,5 bis 10 Gew.% und in einer besonders bevorzugten Ausführungsform von 0,5 bis 6 Gew.% eingesetzt.

Werden die α-Hydroxycarbonsäureester ethoxylierter Alkohole der Komponente (b) in Kombination mit Alkylethersulfaten als Komponente (a) eingesetzt, so hat es sich gezeigt, dass die konditionierende Wirkung dieser Zusammensetzung besonders stark bei einem Mischungsverhältnis von (a) : (b) im Bereich von 1 : 10 bis 10 : 1, insbesondere im Bereich von 1 : 1 bis 10 : 1 ausgeprägt ist.

Es ist davon auszugehen, dass die konditionierenden Eigenschaften der kosmetischen Zubereitungen der vorliegenden Patentanmeldung insbesondere auf die Anwesenheit der Komponente (b) zurückgehen. Daher können die α-Hydroxycarbonsäureester ethoxylierter Alkohole der Formel (I)

R¹O(CH₂CH₂O)nH (I)

in der R¹ für einen linearen oder verzweigten Alkyl- und/oder Alkenylrest mit 6 bis 22 Kohlenstoffatomen und n für Zahlen von 1 bis 50 steht, als Konditioniermittel in kosmetischen Zubereitungen verwendet. werden. Unter Konditionierung wird in diesem Zusammenhang die Beeinflussung des Griffs, der Kämmbarkeit, der Weichheit, des Glanzes und der Glätte von Haut und Haar verstanden. Daher sind weitere Gegenstände der vorliegenden Erfindung die Verwendung von α-Hydroxycarbonsäureester ethoxylierter Alkohole der Formel (I)

R¹O(CH₂CH₂O)ₙH (I)

in der R¹ für einen linearen oder verzweigten Alkyl- und/oder Alkenylrest mit 6 bis 22 Kohlenstoffatomen und n für Zahlen von 1 bis 50 steht, in kosmetischen Zubereitungen zur Verbesserung des Haut- und des Haargriffs/-gefühls, zur Verbesserung der Naß- und Trockenkämmbarkeit und Antistatik von Haaren, der Verbesserung der Weichheit von Haut und Haar, der Erhöhung des Glanzes und/oder der Glätte von Haut und Haar sowie als Moisturizer für Haare. Diese Effekte werden durch die erfindungsgemäßen α-Hydroxycarbonsäureester ethoxylierter Alkohole insbesondere daher hervorgerufen, da sie das Aufziehen von Alkylethersulfaten auf die Haut verhindern oder zumindest vermindern. Daher besteht ein weiterer Gegenstand der vorliegenden Erfindung in der Verwendung von α-Hydroxycarbonsäureestern ethoxylierter Alkohole der Formel (I)

R¹O(CH₂CH₂O)ₙH (I),

in der R1 für einen linearen oder verzweigten Alkyl- oder Alkenylrest mit 6 bis 22 Kohlenstoffatomen und n für Zahlen von 1 bis 50 steht, zur Verhinderung oder Verminderung des Aufziehens von Alkylethersulfaten auf menschliche Haut.

### Erdalkaliionen

Die erfindungsgemäßen Zubereitungen enthalten weiterhin 0,005 bis 1,5 Gew. %, vorzugsweise 0,005 bis 1,0 Gew. %, besonders bevorzugt 0,01 bis 1,0 Gew. % und in einer ganz besonders bevorzugten Ausführungsform 0,01 bis 0,5 Gew.% Alkaliionen. Bevorzugt werden hierbei Calciumionen und/oder Magnesiumionen eingesetzt. Dabei ist zu beachten, dass das zur Herstellung der kosmetischen Zubereitung verwendete Wasser gegebenenfalls die gewünschte Ionenkonzentration bereits beinhaltet. Durch den Einsatz dieser Ionen in den kosmetischen Zubereitungen wird die konditionierende Wirkung der α-Hydroxycarbonsäureester ethoxylierter Alkohole der Formel (I) verstärkt.

### Silikonverbindungen

Die erfindungsgemäßen Zubereitungen enthalten weiterhin 0,1 bis 3 Gew.% einer Silikonverbindung. Hierunter sind insbesondere lösliche oder unlösliche, sowie flüchtige oder nichtflüchtige Polyorganosiloxane zu verstehen. Hierzu gehören Polyalkylsiloxane, Polyarylsiloxane, Polyalkylarylsiloxane, Silikongummis und -harze, sowie chemisch modifizierte Polyorganosiloxane. Unter den Polyalkylsiloxane ist insbesondere das Polydimethylsiloxan mit terminalen CH₃-Gruppen bevorzugt. Die chemisch modifizierten Polyorganosiloxane können hydroxy-, thiol-, acyloxyacyl-, hydroxyacylamino-, amino- oder alkylenoxy-modifiziert sein. Insbesondere die amino- und hydroxyfunktionalisierten Polyorganosiloxane sind im Sinne der vorliegenden Erfindung bevorzugt.

In einer besonders bevorzugten Ausführungsform enthalten die erfindungsgemäßen kosmetischen Zubereitungen
(a) 9 bis 20 Gew. % mindestens eines anionischen, nichtionischen und/oder amphoteren Tensids,
(b) 0,5 bis 6 Gew. % eines α-Hydroxycarbonsäureesters ethoxylierter Alkohole der Formel (I)

   R¹O(CH₂CH₂O)ₙH (I),

   in der R1 für einen linearen oder verzweigten Alkyl- oder Alkenylrest mit 6 bis 22 Kohlenstoffatomen und n für Zahlen von 1 bis 50 steht,
(c) 0,01 bis 0,5 Gew.% mindestens eines Erdalkaliions und
(d) 0,5 bis 3 Gew.% einer Silikonverbindung.

### Hilfs- und Zusatzstoffe

Diese erfindungsgemäßen Zubereitungen können ferner als weitere Hilfs- und Zusatzstoffe Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene und andere Wirkstoffe, UV-Lichtschutzfaktoren, Antioxidantien, Antischuppenmittel, Filmbildner, Quellmittel, Hydrotrope, Solubilisatoren, Konservierungsmittel, Parfümöle, Farbstoffe und dergleichen enthalten.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃₋Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-snglycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Noveon; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid. Weiterhin seien noch Natriumpolynaphthalensulfate, Acrylat/Aminoacrylat/C₁₀₋₃₀-Alkyl PEG-20 Itaconat-Copolymere und Polyacrylamidomethylpropansulfonsäure genannt.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Crinipan® AD (Climbazole), Ketocönazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Noveon) dienen.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Wirkstoffe

Als Wirkstoffe können Proteine und Proteinderivate, wie z.B. Proteinkondensate oder Proteinhydrolysate eingesetzt werden.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotte-öl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Beispiele

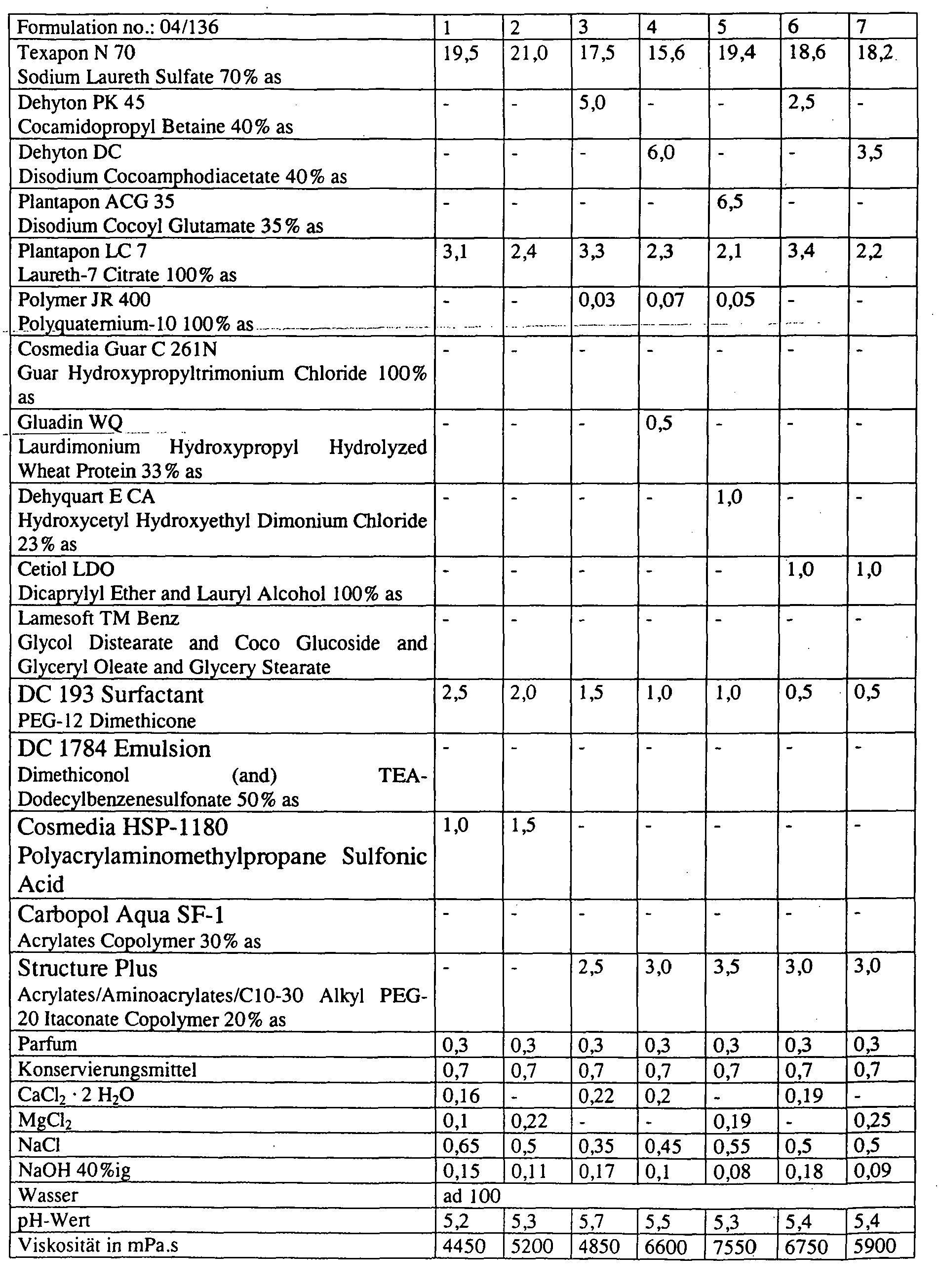

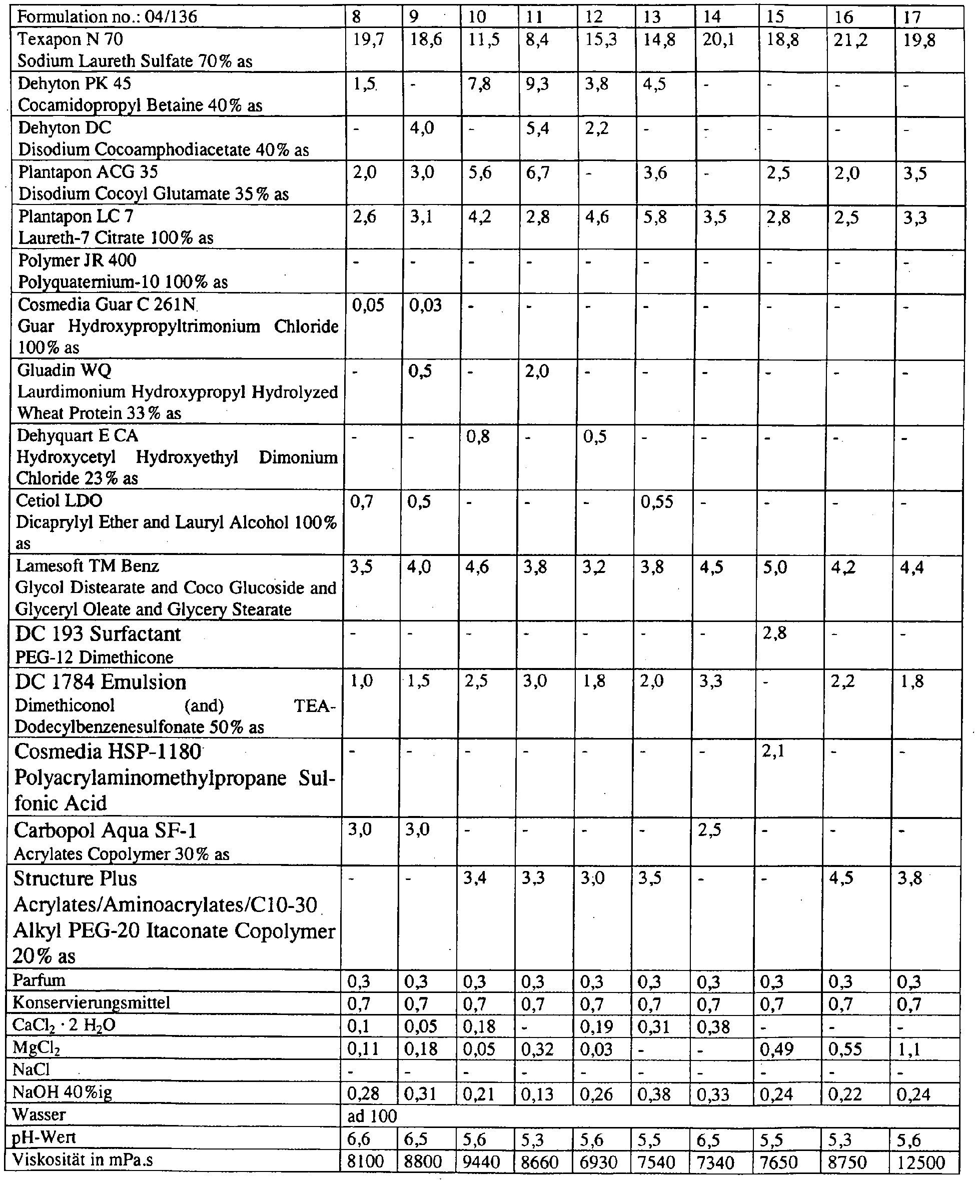

### Sensory Assessment

Mittels Sensory Assessment wurden die konditionierenden Eigenschaften zweier Tensidmischungen miteinander verglichen. Nach dem endgültigen Einziehen der Probenzusammensetzung auf der Haut wurde mit der oberen Seite des Mittel- und Zeigefingers langsam ohne Druck über die behandelte Unterarminnenseite gestrichen. Wurde die Hautoberfläche als ohne Reibung empfunden, entsprach dies einem weichen Hautgefühl.

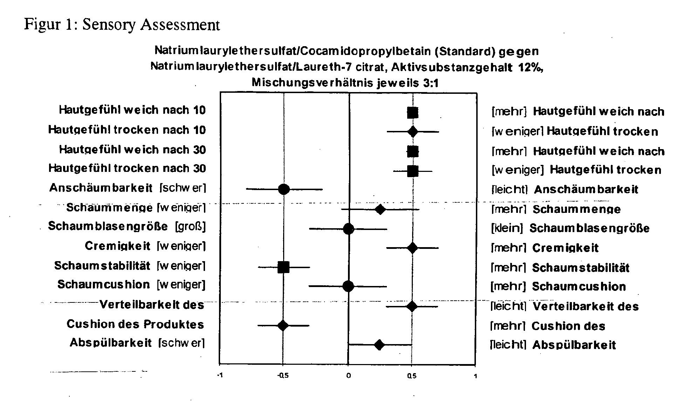

Figur 1 ist zu entnehmen, dass mit Laureth-7 Citrate im Vergleich zu mit Cocamidopropylbetain behandelte Haut als weicher empfunden wird.

### Halbseitentest

Jeder Test wurde an 10 Freiwilligen durch trainiertes und geschultes Fachpersonal durchgeführt. Jede Kopfhälfte wurde vorgewaschen und anschließend mit den entsprechenden Produkten behandelt. Während des Test wurden die Anwendungseigenschaften wie Hautgefühl bewertet, anschließend Parameter wie Kämmbarkeit, Griff, Glanz und Volumen etc.

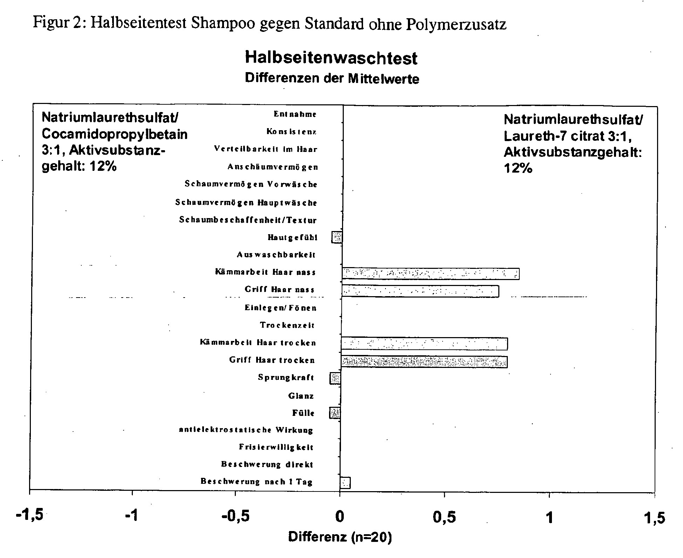

Figur 2 ist zu entnehmen, dass die α-Hydroxycarbonsäureester-haltige Zubereitung gegenüber der Zubereitung, die nur Standardtenside enthält, in bezug auf konditionierende Eigenschaften deutlich überlegen ist. Das Haar läßt sich sowohl im trockenen als auch nassen Zustand deutlich besser kämmen und auch die Griffeigenschaften werden deutlich besser bewertet als bei Behandlung der Haare mit der Standardzubereitung.

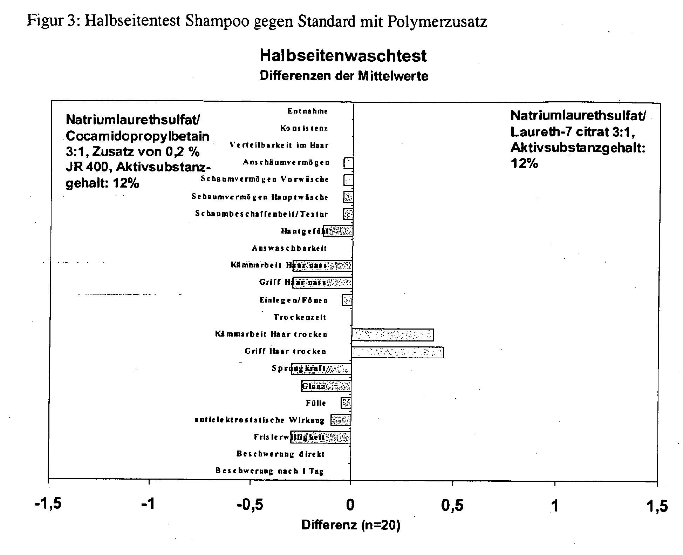

Figur 3 zeigt deutlich auf, dass die α-Hydroxycarbonsäureester-haltige Zubereitung zu einer verbesserten Kämmbarkeit des trockenen Haars, sowie einem angenehmeren Griff des Haares führt.

### Aufziehverhalten

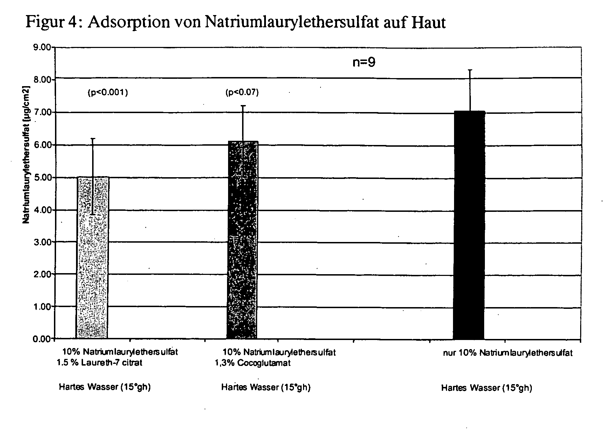

Figur 4 zeigt, dass bei Anwesenheit erfindungsgemäßer α-Hydroxycarbonsäureestern ethoxylierter Alkohole der Formel (I) (Laureth-7 citrat) die Adsorption von Natriumlaurylethersulfat wesentlich besser verhindert wird als mit anderen Cotensiden (Cocoglutamat).

## Patentansprüche

1. Kosmetische Zubereitung enthaltend
(a) 0,1 bis 30 Gew.% mindestens eines anionischen, nichtionischen und/oder amphoteren Tensids,
(b) 0,05 bis 20 Gew.% eines α-Hydroxycarbonsäureesters ethoxylierter Alkohole der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, (c) 0,005 bis 1,5 Gew.% mindestens eines Erdalkaliions und (d) 0,1 bis 3 Gew.% einer Silikonverbindung.

2. Zubereitung gemäß Anspruch 1, **dadurch gekennzeichnet, dass** als Komponente (a) Alkylethersulfate, Alkylamidobetaine, acylierte Aminosäuren, Alk(en)yloligoglykoside, Alkylglucosecarboxylate oder Alkylamphoacetate eingesetzt werden.

3. Zubereitung gemäß wenigstens einem der Ansprüche 1 und/oder 2, **dadurch gekennzeichnet, dass** als Komponente (b) Ester der Citronensäure mit ethoxylierten Alkoholen der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, eingesetzt werden.

4. Zubereitung gemäß einem der Ansprüche 1 bis 3, **dadurch gekennzeichnet, dass** sie als Komponente (c) Calciumionen enthält.

5. Zubereitung gemäß einem der Ansprüche 1 bis 3, **dadurch gekennzeichnet, dass** sie als Komponente (c) Magnesiumionen enthält.

6. Zubereitung gemäß einem der Ansprüche 1 bis 5, **dadurch gekennzeichnet, dass** als Komponente (d) amino- und hydroxyfunktionalisierten Polyorganosiloxane enthält.

7. Verwendung von α-Hydroxycarbonsäureestern ethoxylierter Alkohole der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, als Konditioniermittel in kosmetischen Zubereitungen.

8. Verwendung von α-Hydroxycarbonsäureestern ethoxylierter Alkohole der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, in kosmetischen Zubereitungen zur Verbesserung des Haut- und des Haargriffes/-gefühls.

9. Verwendung von α-Hydroxycarbonsäureestern ethoxylierter Alkohole der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, in kosmetischen Zubereitungen zur Verbesserung der Naß- und Trockenkämmbarkeit und der Antistatik von Haaren.

10. Verwendung von α-Hydroxycarbonsäureestern ethoxylierter Alkohole der Formel (I)
R¹O(CH₂CH₂O)ₙH (I)
in der R1 für einen linearen oder verzweigten Alkyl- oder Alkenylrest mit 6 bis 22 Kohlenstoffatomen und n für Zahlen von 1 bis 50 steht, in kosmetischen Zubereitungen zur Verbesserung der Weichheit von Haut und Haar.

11. Verwendung von α-Hydroxycarbonsäureestern ethoxylierter Alkohole der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, in kosmetischen Zubereitungen zur Erhöhung des Glanzes und/oder der Glätte von Haut und Haar.

12. Verwendung von α-Hydroxycarbonsäureestern ethoxylierter Alkohole der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, in kosmetischen Zubereitungen als Moisturizer für Haare.

13. Verwendung von α-Hydroxycarbonsäureestern ethoxylierter Alkohole der Formel (I)
R¹O(CH₂CH₂O)ₙH (I),
in der R1 für einen linearen oder verzweigten Alkyl- oder Alkenylrest mit 6 bis 22 Kohlenstoffatomen und n für Zahlen von 1 bis 50 steht, zur Verhinderung oder Verminderung des Aufziehens von Alkylethersulfaten auf menschliche Haut.
